# EUROPÄISCHE PATENTANMELDUNG

(11) **EP 3 173 729 A1**
(43) Veröffentlichungstag der Anmeldung: **31.05.2017**
(21) Anmeldenummer: 16002428.7
(22) Anmeldetag: 16.11.2016
(51) Int. Cl.: F41H 13/00, F42B 8/26, F42B 12/42, G02C 7/10, A61F 9/06, A61F 9/02

(54) **LICHTOPTISCHE IRRITATIONSEINRICHTUNG**

(30) Priorität: 26.11.2015 DE 102015015339
(71) Anmelder: Diehl Defence GmbH & Co. KG, 88662 ÜBERLINGEN (DE)
(72) Erfinder: Stark, Robert, DE - 91438 Bad Windsheim (DE)
(74) Vertreter: Diehl Patentabteilung

(57) **Zusammenfassung**

Lichtoptische Irritationseinrichtung, umfassend eine Blendeinrichtung mit einem oder mehreren Leuchtmitteln und eine Steuerungseinrichtung, wobei das oder die Leuchtmittel zur Emission eines oder mehrerer Lichtpulse mit einer bekannten Frequenz oder einem bekannten Frequenzband ansteuerbar ist oder sind, sowie wenigstens eine Schutzeinrichtung, die von einer durch den Lichtpuls irritierbaren Person tragbar oder an einer durch den Lichtpuls irritierbaren Gerätschaft anbringbar oder integriert ist, und die eine Filtereinrichtung, die auf die Frequenz oder das Frequenzband des Lichtpulses und/oder die Gabefrequenz der Lichtpulse abgestimmt ist, umfasst.

## Beschreibung

Die Erfindung betrifft eine lichtoptische Irritationseinrichtung, umfassend eine Blendeinrichtung mit einem oder mehreren Leuchtmitteln und eine Steuerungseinrichtung, wobei das oder die Leuchtmittel zur Emission eines oder mehrerer Lichtpulse mit einer bekannten Frequenz oder einem bekannten Frequenzband ansteuerbar ist oder sind.

Eine solche lichtoptische Irritationseinrichtung dient der kurzzeitigen Blendung und Irritation von Personen oder optischen Geräten, indem ein kurzzeitiger, extrem heller Lichtblitz, also ein Lichtpuls hoher Intensität, erzeugt wird, der im Umfeld stehende Personen blendet respektive optische Geräte, die mit optischen Sensoren arbeiten, zu temporären Fehlmessungen verleitet. Der Lichtpuls oder Lichtblitz wird mittels einer Blendeinrichtung, die ein oder mehrere in oder an einem Gehäuse angeordnete Leuchtmittel umfasst, erzeugt. Ein solches Leuchtmittel kann beispielsweise eine LED sein. Diese emittiert Licht mit einer bekannten Frequenz oder einem bekannten Frequenzband. Die Steuerung des Betriebs des oder der Leuchtmittel erfolgt über eine blendeinrichtungsseitige vorgesehene Steuerungseinrichtung. Eine solche Blendeinrichtung wird beispielsweise in Form einer Blendgranate verwendet, die von Hand verbracht oder verschossen werden kann.

Wenngleich mit einer solchen Irritationseinrichtung respektive Blendeinrichtung eine Irritation des Gegners oder gegnerischer optischer Gerätschaften erreichbar ist, besteht das Problem, dass die Blendeinrichtung den Lichtblitz oder Lichtpuls nicht unbedingt richtungsselektiv emittiert, so dass die Gefahr besteht, dass auch eigenes Personal oder eigene optische Gerätschaften von dem emittierten Lichtpuls mehr oder weniger stark irritiert werden können.

Der Erfindung liegt damit das Problem zugrunde, eine lichtoptische Irritationseinrichtung anzugeben, die diesbezüglich Abhilfe schafft.

Zur Lösung dieses Problems ist eine lichtoptische Irritationseinrichtung umfassend die beschriebene Blendeinrichtung vorgesehen, wobei die lichtoptische Irritationseinrichtung des Weiteren wenigstens eine Schutzeinrichtung umfasst, die von einer durch den Lichtpuls irritierbaren Person tragbar oder an einer durch den Lichtpuls irritierbaren Gerätschaft anbringbar oder integriert ist, und die eine Filtereinrichtung, die auf die Frequenz oder das Frequenzband des Lichtpulses und/oder die Gabefrequenz der Lichtpulse abgestimmt ist, umfasst.

Die erfindungsgemäße Irritationseinrichtung zeichnet sich durch eine zusätzliche Schutzeinrichtung aus, die eine Person, die die Schutzeinrichtung trägt, oder eine Gerätschaft, an der diese Schutzeinrichtung angebracht oder integriert ist, vor einer Irritation durch den emittierten Lichtpuls schützt. Dies gelingt dadurch, dass die Schutzeinrichtung eine Filtereinrichtung aufweist, die in der Lage ist, Licht mit der Frequenz oder dem Frequenzband, mit der oder dem der Lichtpuls erzeugt wird, herauszufiltern. Das heißt, dass die Filterfrequenz oder das Filterfrequenzband der Filtereinrichtung auf die Frequenz oder das Frequenzband des Lichtpulses abgestimmt ist. Dies ist möglich, nachdem wie beschrieben das oder die Leuchtmittel den Lichtpuls mit einer bekannten Frequenz oder einem bekannten Frequenzband emittieren, so dass die Filtereinrichtung entsprechend eingerichtet respektive eingestellt werden kann.

Hierüber wird erreicht, dass zum einen die Blendeinrichtung ohne weiteres verbracht werden kann, ohne dass in irgendeiner Weise auf eine besondere Positionierung oder Ausrichtung oder dergleichen zu achten ist. Zum anderen wird das eigene Personal respektive werden eigene Gerätschaften vor einer Irritation durch den erzeugten Lichtpuls sicher geschützt, so dass entsprechende Beeinträchtigungen ausgeschlossen sind.

Die Frequenz oder das Frequenzband des Lichtpulses und damit auch die Filterfrequenz oder das Filterfrequenzband der Filtereinrichtung sollte im Bereich von 280 nm bis 1 mm liegen. Bevorzugt ist das Frequenzband sehr schmal, so dass die Filtereinrichtung nur ein schmalbandiges Filterfrequenzband aufweist, mithin also Licht mit anderen Wellenlängen respektive Frequenzen, insbesondere Arbeitsfrequenzen der optischen Gerätschaft durchlässt. Zur Personenirritation sollte die Frequenz oder das Frequenzband im Bereich des sichtbaren Lichts liegen. Auch viele optische Gerätschaften können mit einem Lichtpuls aus diesem Spektralbereich irritiert werden. Optische Gerätschaften arbeiten häufig aber auch unter Verwendung von Infrarotsensoren, weshalb der Lichtpuls auch mit einer Frequenz oder einem Frequenzband im Infrarotbereich emittiert werden kann. Auch kann der emittierte Spektralbereich den UV-Bereich umfassen, wenn dies der Einsatzzweck erfordert. Selbstverständlich ist es denkbar, dass der Lichtpuls Licht mit mehreren unterschiedlichen Frequenzen oder Frequenzbändern umfasst, so beispielsweise einer Frequenz oder ein Frequenzband aus dem Bereich des sichtbaren Lichts und einer Frequenz oder einem Frequenzband aus dem Infrarotbereich, um eine sichere Irritation sowohl von Personen als auch von Gerätschaften mit einem einzigen Lichtpuls zu ermöglichen.

Als Leuchtmittel, die die Emission von Licht mit einer bekannten Frequenz oder einem schmalen Frequenzband ermöglichen, werden bevorzugt solche auf Halbleiterbasis verwendet, wobei sich hierfür LEDs oder insbesondere Laserdioden oder Laser eignen. Insbesondere Laserquellen wie z. B. die beschriebenen Laserdioden zeichnen sich dadurch aus, dass sie monochromatisches, kohärentes Licht erzeugen. Durch die Verwendung mehrerer solcher Laserlichtquellen, die Licht mit unterschiedlichen Frequenzen emittieren, kann die Blendeinrichtung, je nachdem, welche Laserlichtquelle angesteuert wird, entweder einen monofrequenten Lichtpuls oder einen multifrequenten Lichtpuls, der also Licht mehrerer definierter Laserfrequenzen umfasst, abstrahlen. Dies ist ohne weiteres möglich, als der Betrieb der Blendeinrichtung über die Steuerungseinrichtung vollelektronisch gesteuert ist. Da die jeweilige Frequenz bekannt ist, kann die Schutzeinrichtung ohne weiteres entsprechend eingerichtet werden und folglich frequenzselektiv arbeiten. Ein Beispiel für solche Schutzeinrichtungen sind beispielsweise Schutzbrillen, Shutterbrillen oder spezifische Gläser oder Filter sowie die Verwendung von elektronisch gesteuerten Filtern und dergleichen, wobei die Schutzeinrichtungen auch polarisationsselektiv sein können.

Zweckmäßig ist es, wenn die Leuchtmittel jeweils drei LEDs oder Laserdioden umfassende RGB-Cluster sind. Das heißt, dass drei unterschiedliches Licht emittierende LEDs oder Laserdioden zu einem sogenannten RGB-Cluster (RGB = Rot, Grün, Blau) kombiniert werden, wobei die einzelnen kombinierten LEDs/Laserdioden in diesem Fall rotes, grünes und blaues Licht emittieren. Durch entsprechende simultane Ansteuerung ergibt sich somit durch Farbmischung ein entsprechendes Licht, das auch hinreichend schmalbandig erzeugt werden kann. Solche RGB-Cluster sind als fertige Bauteile bereits verfügbar. Sie haben darüber hinaus den Vorteil, dass bei entsprechender Auslegung der Steuerungseinrichtung die einzelnen Farbkomponenten des gemischten Lichtes unterschiedlich gewichtet werden können, so dass folglich nahezu beliebige Frequenzen gemischt werden können. Insbesondere die Verwendung von Laserdioden innerhalb eines solchen RGB-Clusters ist im Hinblick auf die Erzeugung eindeutiger respektive schmalbandiger Frequenzbänder zweckmäßig, da wie beschrieben solche Laserdioden monochromes und kohärentes Licht emittieren.

Gemäß einer zweckmäßigen Weiterbildung der Erfindung kann vorgesehen sein, dass mehrere Lichtpulse hintereinander emittierbar sind, wobei die Pulsgabefrequenz und/oder die Pulsdauer einstellbar ist. Es ist also möglich, nur einen einzelnen Lichtpuls zu erzeugen, oder mehrere kurzzeitig hintereinander abfolgende Pulse, oder ein Burst an Pulsen, wobei die Frequenz, mit der die Pulse hintereinander gegeben werden, oder die Dauer, wie lange ein solcher Puls gegeben wird, und die Pulsanzahl einstellbar ist. Es ist also eine beliebige Modulationsmöglichkeit gegeben, die Pulsgabe regelmäßig oder unregelmäßig zu gestalten. Je nach Ausgestaltung der entsprechenden Schutzeinrichtung kann diese lediglich auf die Frequenz abgestimmt sein, insbesondere bei elektronisch einstellbaren Schutzeinrichtungen kann gleichzeitig auch eine Synchronisation bezüglich der zeitlichen Pulsabgabefolge respektive der Pulsdauer erfolgen, so dass die Filterwirkung nur dann gegeben ist, wenn tatsächlich ein Lichtpuls gegeben wird.

Wie bereits beschrieben ist es bei Verwendung mehrerer Leuchtmittel zweckmäßig, wenn die einzelnen Leuchtmittel Licht mit zueinander unterschiedlichen Frequenzen oder Frequenzbändern emittieren, das heißt, dass unterschiedliche LEDs oder Laserdioden verwendet werden. Die Frequenz oder das Frequenzband des Lichtpulses kann fest vorgegeben sein, das heißt, dass bei Erzeugung eines Lichtpulses stets Licht mit einer definierten, unveränderbaren Frequenz oder einem entsprechenden Frequenzband emittiert wird. Alternativ kann, wie beschrieben, die Frequenz oder das Frequenzband einstellbar sein, was insbesondere bei Verwendung der bereits beschriebenen RGB-Cluster ohne weiteres möglich ist. Die Frequenzänderung ist dahingehend von Vorteil, als gegnerischerseits eine solche Änderung, die auch in situ, also unmittelbar vor Ort eingestellt respektive programmiert werden kann, nicht nachvollziehbar ist. Sie ist jedoch seitens der eigenen Schutzeinrichtungen ohne weiteres nachvollziehbar, da wie beschrieben die emittierten Lichtfrequenzen bekannt sind. Eine entsprechende Nachführung ist wie beschrieben insbesondere bei elektronisch einstellbaren Filtern, die ohne weiteres mit der Blendeinrichtung respektive deren Steuerungseinrichtung synchronisiert werden können, möglich. Das heißt, dass in einem solchen Fall auch die Filterfrequenz oder das Filterfrequenzband einstellbar ist, so dass sich entsprechende Änderungen sofort nachvollziehen lassen und das eigene Personal sowie die eigenen Gerätschaften bestmöglich auch bei sich ändernden Frequenzen geschützt sind.

Alternativ dazu kann die Filterfrequenz oder das Filterfrequenzband auch fest vorgegeben sein, was beispielsweise bei Verwendung einfacher Schutzbrillen mit Filtergläsern oder bei Kamerafilterscheiben, die auf definierte Frequenzen oder Frequenzbänder abgestimmt sind, der Fall ist. In diesem Fall sind die Schutzeinrichtungen bevorzugt hinsichtlich des Filterfrequenzbandes breiter auszulegen, so dass eine Änderung der Frequenz oder des Frequenzbandes des Lichtpuls in gewissen Grenzen möglich ist, gleichzeitig aber auch ein entsprechender Schutz seitens der Schutzeinrichtung gegeben ist.

Die Schutzeinrichtung selbst ist bevorzugt eine elektronisch steuerbare Schutzeinrichtung respektive weist eine elektronisch steuerbare Filtereinrichtung auf. Hierunter sind z. B. elektronisch gesteuerte Blenden zu verstehen, die z. B. in Abhängigkeit der Gabefrequenz der Lichtpulse öffnen und schließen, oder bei Gabe nur eines Lichtpulses geschlossen werden. Alternativ sind Polarisationsfilter denkbar, wie auch LCD-Filter, wobei diese Aufzählung nicht abschließend ist. Grundsätzlich ist jedwede elektronisch steuerbare Filtereinrichtung verwendbar, die sich hinsichtlich der Filterfrequenz elektronisch verändern lässt oder hinsichtlich der Pulsgabefrequenz die elektronisch gesteuert geöffnet und geschlossen werden kann.

Zur Abstimmung der elektronisch steuerbaren Filtereinrichtung auf die Frequenz oder das Frequenzband eines zu gebenden Lichtpulses oder eine Pulsfolge ist die steuerbare Filtereinrichtung und die Steuerungseinrichtung der Blendeinrichtung bevorzugt miteinander synchronisiert bzw. synchronisierbar, so dass jedwede Änderung ohne weiteres nachvollzogen werden kann, sei es hinsichtlich des Frequenzgangs, der Pulsfolge, der Polarisation oder einer Änderung der Frequenzen. Bevorzugt kommunizieren die Schutzeinrichtungen und die Blendeinrichtung diesbezüglich drahtlos miteinander, so dass die Synchronisation auch über weitere Strecken ohne weiteres möglich ist. Alternativ dazu kann, insbesondere wenn die Blendeinrichtung, worauf nachfolgend noch eingegangen wird, an einem Kraftfahrzeug verbaut ist, auch eine drahtgebundene Kommunikation insbesondere zu kraftfahrzeugseitigen Gerätschaften vorgesehen sein.

Bei der Blendeinrichtung kann es sich, wie bereits beschrieben, um eine Blendgranate handeln, die ein entsprechendes, relativ kleinformatiges Gehäuse aufweist, an oder in dem das oder die Leuchtmittel, bevorzugt die Laserdioden, nebst Steuerungseinrichtung und gegebenenfalls einem, vorzugsweise wiederaufladbaren, Energiespeicher vorgesehen sind. Diese Blendgranate ist hinreichend klein, sie kann also ohne weiteres von Hand verbracht werden. Das Gehäuse kann eine rundliche, also quasi zylindrische Geometrie, eine kugelförmige Geometrie, eine kubische Geometrie oder dergleichen aufweisen. Alternativ dazu kann die Blendeinrichtung auch eine lineare oder bogenförmige oder ringförmige Geometrie aufweisen. Eine lineare Blendeinrichtung kann beispielsweise als länglicher Stab ausgeführt sein, an dem ein oder mehrere Leuchtmittel angeordnet sind. Mit einer solchen Blendeinrichtung kann beispielsweise ein Lichtvorhang generiert werden. Eine solche Blendeinrichtung kann beispielsweise an einem Fahrzeug außenseitig angebracht werden. Eine bogen- oder ringförmige Geometrie kann beispielsweise eine an einem UAV (UAV = Unmaned Aerial Vehicle) angeordnete Einrichtung aufweisen. Mit dieser ist es möglich, von dem UAV aus die Blendwirkung zu erzeugen, indem das UAV in den Bereich, wo der Lichtpuls generiert werden soll, geflogen wird. Soweit es sich bei der Blendeinrichtung um eine mobile, also von einem Versorgungsfahrzeug extern arbeitende Blendeinrichtung handelt, weist diese bevorzugt einen integrierten Energiespeicher auf. Eine an einem Kraftfahrzeug angeordnete Blendeinrichtung kann beispielsweise über eine Kabelverbindung direkt an die Fahrzeugbatterie angeschlossen und über diese versorgt werden.

Die Leuchtmittel selbst sind bevorzugt in Form separater Leuchtmittel-Arrays vorgesehen, wobei an oder in einem Gehäuse der Blendeinrichtung mehrere solcher Arrays verteilt angeordnet sind, die entsprechend separat oder simultan über die Steuerungseinrichtung ansteuerbar sind, um Pulsvariationen und dergleichen zu erzeugen. Auch können den Leuchtmitteln, insbesondere den Arrays, optische Elemente zur Beeinflussung des emittierten Lichts zugeordnet sein, beispielsweise Linsen, Prismen oder feste oder bewegliche Spiegel. Je nach verwendetem Element ist es möglich, eine Strahlaufweitung, eine Strahlsplittung, eine Strahlfokussierung oder eine Strahlformung vorzunehmen, mithin also in irgendeiner Form auf die räumliche Lichtverteilung einzuwirken. Folgt beispielsweise über eine Linse eine entsprechende Strahlaufweitung, so kann der Bereich, der mit dem von einem Leuchtmittel oder einem Array emittierten Licht erfasst wird, deutlich erweitert werden. Dies ist insbesondere von Vorteil, wenn am Gehäuse eine Vielzahl solcher Leuchtmittel oder Arrays vorgesehen sind, so dass durch entsprechende Strahlaufweitung und entsprechende Überlagerung der einzelnen Lichtemissionen der emittierte Lichtpuls in einen extrem großen Raumwinkelbereich emittiert wird und es zu einer umfänglichen räumlichen Irritationswirkung kommt. Alternativ können hierdurch auch die einzelnen Lichtpulse der separaten Leuchtmittel oder Arrays gebündelt werden, um einen gerichteten, also räumlich begrenzten Lichtpuls zu generieren, beispielsweise im Rahmen einer Interferenz bei RGB-Clustern und Ähnlichem.

Die Leuchtmittel, insbesondere die Arrays, sind bevorzugt an einem Reflektor angeordnet, der eine hohe Lichtausbeute sicherstellt, gleichzeitig aber auch eine gewisse Strahlführung ermöglicht. Er kann napfartig sein, so dass die Leuchtmittel respektive Arrays am Reflektorboden aufsitzen. Er kann querschnittlich rechteckig sein, wobei aber auch andere Reflektorgeometrien denkbar sind. Bevorzugt ist hierbei der Reflektor über ein optisches Element, insbesondere eine Linse, geschlossen, so dass sich eine kompakte Baueinheit ergibt, die dann gehäuseseitig direkt verbaut werden kann.

Das oder die Leuchtmittel respektive Arrays können über die Steuerungseinrichtung in einem programmierbaren Muster angesteuert werden. Die Steuerungseinrichtung, eine entsprechend programmierte CPU, kann mit beliebigen Steuerprogrammen belegt werden, über die die Ansteuerung der einzelnen Leuchtmittel respektive Arrays erfolgt. Hierüber ist eine synchrone Ansteuerung aller Leuchtmittel möglich, um zeitlich synchronisierte Lichtpulse zu erzeugen. Auch können einzelne Leuchtmittel in einer bestimmten Zeitabfolge angesteuert werden, wie auch beliebige Gruppen zeitlich nacheinander angesteuert werden können. Es sind unterschiedliche Wiederholungsraten respektive unterschiedliche Wiederholungszeiten definierbar, wie auch Intensitätsvariationen möglich sind. Die Lichtemission kann also quasi beliebig gesteuert werden, sie kann dauerhaft sein, zeitlich begrenzt, regelmäßig, unregelmäßig oder moduliert erfolgen.

Ferner kann die Steuerungseinrichtung einen, gegebenenfalls bedienerseitig einstellbaren, Timer umfassen. Über diesen Timer kann eine definierte Verzögerung vorgegeben werden, die entweder fest vorgegeben ist, das heißt, dass der Timer automatisch anläuft, wenn die Blendeinrichtung aktiviert wird. Sie kann aber auch gegebenenfalls eingestellt werden, wozu beispielsweise ein entsprechendes Bedienelement am Gehäuse vorgesehen ist, um die Steuerungseinrichtung bzw. den Timer zu programmieren. Eine definierte Verzögerung ist abhängig vom Einsatzzweck mitunter erforderlich. Auch diese Timereinstellung kann ohne weiteres über eine entsprechende Synchronisation mit der Filtereinrichtung dortseits nachvollzogen werden, soweit es sich um eine einstellbare Filtereinrichtung handelt.

Weiterhin kann am oder im Gehäuse ein Abstandssensor zur Erfassung des Abstands der Blendeinrichtung zu einem benachbarten Gegenstand vorgesehen sein, wobei die Steuerungseinrichtung die Leuchtmittel in Abhängigkeit der Abstandsinformation ansteuert. Dies ermöglicht es, die "Zündung", also die Lichtpulsemission, nur dann vorzunehmen, wenn tatsächlich eine zu irritierende Person respektive Gerätschaft in der Nähe ist. Die Blendeinrichtung kann also bereits vorher deponiert werden, agiert jedoch nur dann, wenn sich tatsächlich eine entsprechende Situation ergibt. Nähert sich die Person oder die Gerätschaft in einem gegebenenfalls parametrierbaren Mindestabstand zur Blendeinrichtung, erfolgt die Zündung. Auch dieser unbekannte Zeitpunkt kann über die Synchronisation seitens der elektronisch steuerbaren Schutzeinrichtung ohne weiteres erfasst werden, die dann zum exakten Zeitpunkt den Schutz bietet.

Eine weitere vorteilhafte Ausgestaltung der Irritationseinrichtung sieht vor, dass im Gehäuse der Blendeinrichtung eine Kommunikationseinrichtung umfassend zumindest einen mit der Steuerungseinrichtung kommunizierenden Empfänger zum Empfangen von von einem externen Sender gegebenen Steuersignalen oder Programmierinformationen vorgesehen ist. Die Kommunikationseinrichtung ermöglicht eine Fernsteuerung der Blendeinrichtung über eine externe Fernsteuereinrichtung. So ist es beispielsweise möglich, die Blendeinrichtung zu verbringen oder aus gesicherter Distanz zu beobachten, ob sich ein Szenario ergibt, das das Zünden der Blendgranate erfordert. Wird dies festgestellt, kann über einen entsprechenden Fernsteuerungssender ein entsprechendes Signal übertragen werden, das von der Kommunikationseinrichtung empfangen wird, woraufhin die Zündung erfolgt. Auch kann hierüber ein entsprechendes Steuerprogramm ausgewählt werden, wenn nach einer Analyse des gegebenen Szenarios klar ist, wie die Blendwirkung erfolgen soll, das heißt, welches Blendmuster, welche Intensität und welche Frequenz etc. gewünscht wird.

In Weiterbildung der Erfindung kann die Kommunikationseinrichtung ferner auch einen mit der Steuerungseinrichtung kommunizierenden Sender zum Aussenden von Signalen umfassen. Dieser Sender ermöglicht es, dass einerseits die blendeinrichtungsseitige Steuerungseinrichtung mit einer externen Steuerungseinrichtung in bidirektionale Kommunikation treten kann. Andererseits können hierüber auch entsprechende Synchronisationsdaten an die Schutzeinrichtung gegeben werden. Auch kann eine Blendeinrichtung mit einer anderen diesbezüglich kommunizieren und sich synchronisieren, so dass ein Blendsystem aufgebaut werden kann, in dem nicht nur einzelne Blendeinrichtungen mit beliebigen programmierbaren Arbeitsmustern arbeiten, sondern in dem auch die Blendeinrichtungen untereinander zeitlich verschieden betrieben werden. Ein Master-Slave-Betrieb in Verbindung mit einer übergeordneten Fernsteuerungseinrichtung ist hierüber ohne weiteres realisierbar, verbunden mit einer stets gegebenen Abstimmung respektive Synchronisation der zugeordneten Schutzeinrichtungen.

Bevorzugt ist, wie bereits beschrieben, im Gehäuseinneren ein wiederaufladbarer Energiespeicher vorgesehen, der dem Gehäuse entnehmbar ist, oder der über einen Ladeanschluss am Gehäuse aufgeladen werden kann. Ferner kann ein den Betrieb freischaltender, bedienerseitig zu betätigender Schalter vorgesehen sein.

Schließlich kann im Gehäuse eine über die Steuerungseinrichtung ansteuerbare Selbstzerstörungseinrichtung, insbesondere durch Zerstörung der Steuerungseinrichtung, vorgesehen sein.

Weitere Vorteile und Einzelheiten der Erfindung ergeben sich aus den im Folgenden beschriebenen Ausführungsbeispielen sowie anhand der Zeichnungen. Dabei zeigen:
- Fig. 1: eine Prinzipdarstellung einer Irritationseinrichtung,
- Fig. 2: eine Prinzipdarstellung einer Blendeinrichtung in Form einer Blendgranate, hier exemplarisch in kubischer Form,
- Fig. 3: eine vergrößerte Prinzipdarstellung zur Erläuterung der im Inneren der Blendeinrichtung verbauten Betriebselektronik nebst einiger Leuchtmittel und zugeordneten Schutzeinrichtungen,
- Fig. 4: eine Aufsicht auf ein Leuchtmittel in Form eines LED-Arrays, angeordnet in einem Reflektor,
- Fig. 5: eine Seitenansicht der Anordnung aus Fig. 4 mit zwei vergrößerten Array-Detailansichten,
- Fig. 6: eine Prinzipdarstellung einer Blendgranate in Ring- oder Kugelform, und
- Fig. 7: eine Prinzipdarstellung eines Kraftfahrzeugs mit einer Blendeinrichtung in Form einer Leiste.

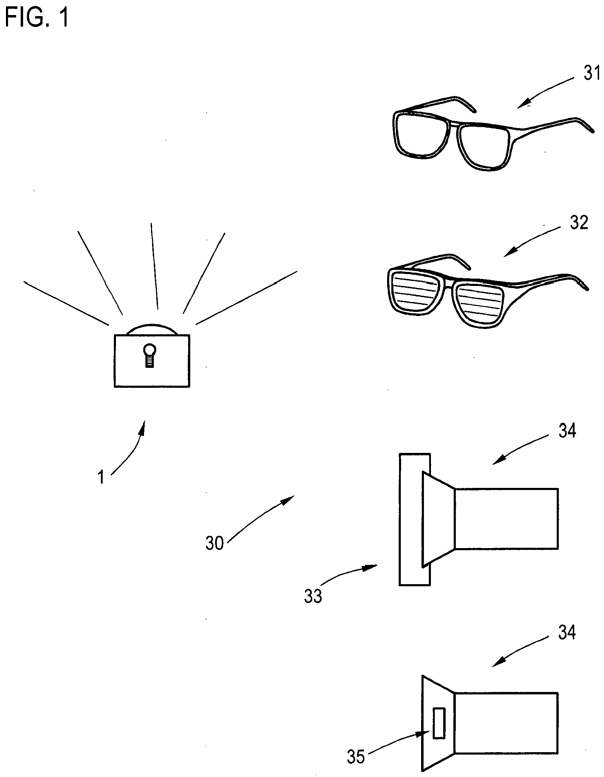

Fig. 1 zeigt in Form einer Prinzipdarstellung eine erfindungsgemäße lichtoptische Irritationseinrichtung. Sie umfasst eine Blendeinrichtung 1, in der ein oder mehrere Leuchtmittel zur Emission eines oder mehrerer kurzzeitiger Lichtpulse vorgesehen sind, die über eine Steuerungseinrichtung entsprechend ansteuerbar sind. Bei den Leuchtmitteln handelt es sich um LEDs oder Laserdioden, die Licht mit einer bekannten Frequenz oder einem bekannten Frequenzband emittieren. Auf den Aufbau einer solchen Blendeinrichtung wird nachfolgend noch detailliert eingegangen.

Der Blendeinrichtung 1 zugeordnet ist wenigstens eine Schutzeinrichtung 30, die dem Schutz einer Person oder einer Gerätschaft vor dem erzeugten Lichtpuls dient. Bei einer solchen Schutzeinrichtung 30 kann es sich beispielsweise um eine Schutzbrille 31 handeln, deren Gläser gerade die Frequenz oder das Frequenzband herausfiltern, mit der oder dem der Lichtpuls erzeugt wird. Eine die Schutzbrille tragende Person wird somit durch den Lichtblitz nicht irritiert.

Alternativ kann die Schutzeinrichtung 30 auch eine Shutterbrille 32 sein, die eine Shutterfunktion aufweist, bei der also die Durchsichtigkeit über die Shuttereinrichtung in hoher Frequenz verändert werden kann. Diese Shutterbrille 32 ist bezüglich des Shutterbetriebs mit der Blendeinrichtung 1 synchronisiert, so dass die Schutzbrille genau dann intransparent wird, wenn der Lichtpuls erzeugt wird. Es handelt sich also um eine elektronisch gesteuerte Filtereinrichtung, die an der Shutterbrille vorgesehen ist.

Ferner ist als Schutzeinrichtung 30 ein Filter 33 gezeigt, der an einer einen lichtoptischen Sensor aufweisenden Gerätschaft 34, z. B. einer Tageslichtkamera oder einer Nachtsichtkamera angeordnet ist. Dieser Filter 33, also ein entsprechendes Filterglas o.dgl., ist mit seiner Filterfrequenz oder dem Filterfrequenzband auf die Frequenz oder das Frequenzband des Lichtpulses abgestimmt, so dass die Gerätschaft vor dem Lichtpuls geschützt ist. Der Filter wird als Zusatzteil an der Gerätschaft montiert.

Schließlich ist eine weitere Schutzeinrichtung 30 in Form einer elektronischen Schutzeinrichtung 35 gezeigt, die in der Gerätschaft, also z. B. der Tages- oder Nachtsichtkamera integriert ist. Bei dieser Schutzeinrichtung 35 kann es sich z. B. um eine elektronisch gesteuerte Blende handeln, die z. B. in Abhängigkeit der Gabefrequenz der Lichtpulse öffnet und schließt, oder bei Gabe nur eines Lichtpulses geschlossen wird. Alternativ kann es ein Polarisationsfilter sein, oder ein LCD-Filter. Grundsätzlich ist jedwede elektronisch steuerbare Filtereinrichtung verwendbar, die sich hinsichtlich der Filterfrequenz elektronisch verändern lässt oder hinsichtlich der Pulsgabefrequenz elektronisch gesteuert geöffnet und geschlossen werden kann.

Soweit die Filterfrequenz der Schutzeinrichtung nicht verändert werden kann, ist sie so auszulegen, dass sie der bekannten Frequenz bzw. dem bekannten Frequenzband des Lichtpulses entspricht. Ist die Filterfrequenz bzw. das Filterfrequenzband jedoch elektronisch einstellbar, so kann durch entsprechende, vorzugsweise drahtlose Synchronisation zwischen der Blendeinrichtung 1 und der Schutzeinrichtung 30 bzw. deren Filtereinrichtung die Filterfrequenz der gegebenen Lichtpulsfrequenz angepasst werden, sei es hinsichtlich der tatsächlichen gefilterten Frequenz, sei es hinsichtlich der Pulsgabefrequenz.

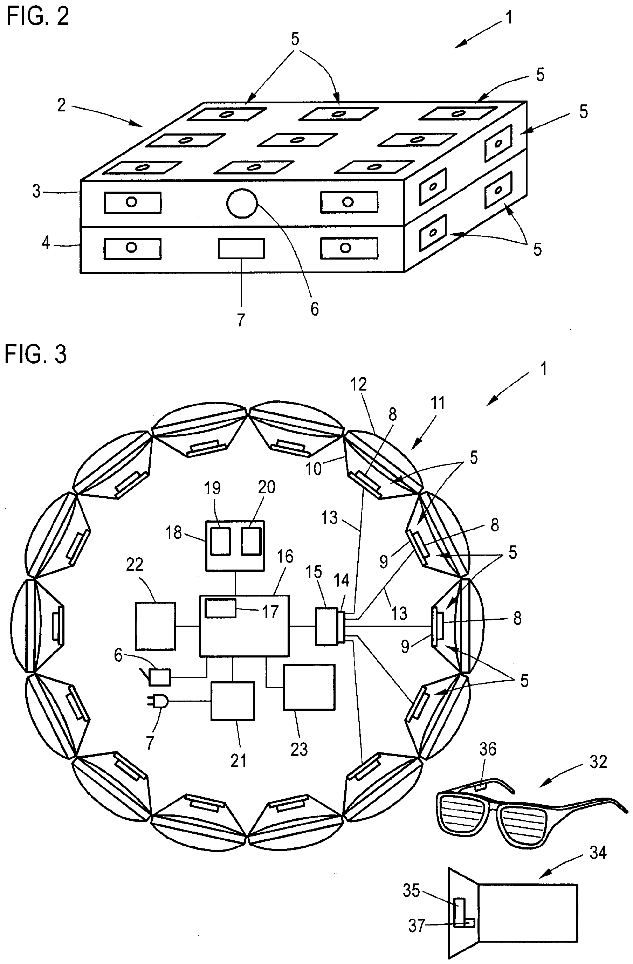

Fig. 2 zeigt eine Blendeinrichtung 1 in Form einer Blendgranate zur Emission eines kurzzeitigen Lichtpulses. Die Blendgranate umfasst ein Gehäuse 2, bestehend aus zwei Halbschalen 3, 4, jeweils vorzugsweise aus Kunststoff. Im gezeigten Beispiel ist das Gehäuse 2 kubisch, es könnte aber gleichermaßen auch kugelförmig sein, das heißt, dass die Halbschalen 3, 4 dann quasi kalottenförmig sind. Am Gehäuse 2 respektive jeder Halbschale 3, 4 sind eine Vielzahl einzelner Leuchtmittel 5 angeordnet, die ihr erzeugtes Licht jeweils nach außen emittieren. Die Leuchtmittel 5 sind, worauf nachfolgend noch eingegangen wird, bevorzugt in Form von Laserdioden-Arrays realisiert und in entsprechenden Reflektoren angeordnet, so dass sich handhabbare Baueinheiten ergeben, die ohne weiteres gehäuseseitig entsprechend verbaut werden können. Am Gehäuse 2 respektive den Halbschalen 3, 4 können entsprechende Vertiefungen respektive Aufnahmen vorgesehen sein, in die jeweils ein Leuchtmittel nebst Reflektor gesetzt wird, so dass die Halbschalenbestückung entsprechend einfach möglich ist. Eine entsprechende Gehäuseausgestaltung ist bei Fertigung aus Kunststoff ohne weiteres möglich.

Im Inneren des Gehäuses 2 wird über die Halbschalen 3, 4 ein entsprechender Hohlraum definiert, in dem die gesamte Betriebselektronik, die nachfolgend noch im Detail geschildert wird, untergebracht ist. An der Gehäuseaußenseite ist im gezeigten Ausführungsbeispiel ein Schalter 6 vorgesehen, bei dem es sich aber auch um ein Tastenfeld handeln kann, über das der Betrieb der Blendeinrichtung 1 aktiviert wird und über das gegebenenfalls ein Zugangscode zur Missbrauchsverhinderung einzugeben ist. Des Weiteren ist exemplarisch ein Ladeanschluss 7 gezeigt, um einen im Gehäuseinneren befindlichen wiederaufladbaren Energiespeicher aufladen zu können, ohne das Gehäuse 2 öffnen zu müssen.

Fig. 3 zeigt in Form einer Prinzipdarstellung eine Blendeinrichtung 1, wobei hier exemplarisch die Leuchtmittel 5 in kreisförmiger Anordnung gezeigt sind. Anstelle eines rechteckigen Gehäuses, wie in Fig. 1 gezeigt, ist selbstverständlich auch ein ringförmiges oder kugelförmiges Gehäuse wie beschrieben denkbar, verbunden mit einer entsprechenden geometrischen Anordnung der Leuchtmittel 5.

Jedes Leuchtmittel 5 besteht wie beschrieben aus einem Laserdioden-Array 8, das exemplarisch auf einem entsprechenden Träger 9 angeordnet ist. Das Laserdioden-Array befindet sich in einem Reflektor 10, der napfartig ist und der oberseitig über ein optisches Element 11, hier eine Linse 12, abgeschlossen ist. Die Linse 12 befindet sich folglich unmittelbar oberhalb des Laserdieoden-Arrays 8 und beeinflusst folglich den vom Laserdioden-Array 8 emittierten Lichtpuls. Die Versorgungsleitungen 13 mehrerer oder aller an einer jeweiligen Halbschale 3, 4 verbauten Leuchtmittel 5 sind beispielsweise in einem Steckverbinder 14 zusammengeführt, so dass sie nicht einzeln zu verbinden sind. Im Beispiel sind nur einige Leuchtmittel zusammengeführt, natürlich sind alle Leuchtmittel 5 entsprechend verschaltet. Der Steckverbinder 14 ist mit einem Laserdioden-Treiber 15 verbunden, der seinerseits mit einer Steuerungseinrichtung 16 verbunden ist, die den gesamten Betrieb der Blendgranate 1 steuert. Die Steuerungseinrichtung 16 verfügt über einen Timer 17, um eine Verzögerung zu definieren, wobei diese Verzögerung entweder vorab eingestellt sein kann und im Rahmen der Aktivierung der Blendeinrichtung 1 angewählt werden kann. Alternativ kann die Verzögerung auch beliebig programmiert werden, beispielsweise per Fernsteuerung. Hierzu ist eine Kommunikationseinrichtung 18 vorgesehen, umfassend einen Empfänger 19 und einen Sender 20. Die Kommunikationseinrichtung 18 kommuniziert mit der Steuerungseinrichtung 16. Über den Empfänger 19 kann die Blendeinrichtung 1 von einer übergeordneten Steuerungseinrichtung, also einer Fernbedingung respektive einem Master, entsprechende Steuer- oder Programmierbefehle erhalten, die dann seitens der Steuerungseinrichtung 16 abgearbeitet werden. In der Steuerungseinrichtung 16 ist in einem geeigneten Speicher eine Mehrzahl an Arbeitsprogrammen abgelegt, mit denen unterschiedliche, von der Programmierung her quasi beliebige Betriebs- bzw. Ansteuermuster für die einzelnen Leuchtmittel 5 hinterlegt sind. Hierüber können die Leuchtmittel allesamt synchron, in zeitlich definierter Abfolge, in Gruppen, mit unterschiedlicher Intensität, oder richtungsselektiv angesteuert werden, so dass letztlich der emittierte Lichtpuls quasi beliebig geformt respektive ausgebildet werden kann. Auch können entsprechende Wiederholraten über das jeweils gewählte Programm definiert werden etc.

Über den Sender 20 besteht die Möglichkeit, dass die Steuerungseinrichtung 16 bidirektional entweder mit einer übergeordneten Fernsteuereinrichtung oder aber mit anderen in der Nähe befindlichen Blendeinrichtungen 1 respektive deren Steuerungseinrichtungen kommuniziert. Hierüber können die verschiedenen Blendgranaten in Betrieb synchronisiert werden, so dass, rein elektronisch gesteuert, die im System eingebundenen verschiedenen Blendeinrichtungen 1 quasi situationsabhängig gesteuert werden können.

Insbesondere kann über den Sender 20 auch die Synchronisation der Blendeinrichtung 1 mit einer Schutzeinrichtung 30 umfassend eine elektronisch einstellbare Filtereinrichtung erfolgen. Sobald seitens der Blendeinrichtung 1 alle Daten betreffend den oder die zu erzeugenden Lichtpulse vorliegen bzw. erfasst sind, können über den Sender 20 an eine oder mehrere externe Schutzeinrichtungen die relevanten Daten, die dortseits zur entsprechenden Einstellung der elektronisch steuerbaren Filtereinrichtung benötigt werden, übertragen werden. Seitens der Schutzeinrichtung werden diese Daten über einen entsprechenden Empfänger empfangen und seitens einer Steuerungseinrichtung verarbeitet, so dass die Filtereinrichtung entsprechend angesteuert bzw. eingestellt werden kann, damit genau die Lichtfrequenz bzw. das Lichtfrequenzband als Filterfrequenz bzw. die Pulsgabefrequenz des oder der zu gebenden Lichtpulse als Shutterfrequenz eingestellt werden kann. Exemplarisch ist in Fig. 3 eine Shutterbrille 32 gezeigt, die über eine Steuerelektronik 36 verfügt, die einen Empfänger zum Empfangen der Synchronisationsdaten und eine Steuerungseinrichtung zum Steuern des Shutterbetriebs in Abhängigkeit der Synchronisationsdaten umfasst. Alternativ ist eine Gerätschaft 34, z. B. eine Kamera gezeigt, die eine integrierte elektronisch steuerbare Schutzeinrichtung 35 umfasst, der ein Empfänger 37 zugeordnet ist, über den die Synchronisationsdaten erfasst werden. Eine in der elektronischen Schutzeinrichtung integrierte oder dieser zugeordnete Steuereinrichtung verarbeitet die Daten und steuert die Schutzeinrichtung 35 bzw. deren Filtereinrichtung entsprechend. Es ist also zumindest ein unidirektionaler Datentransfer gegeben. Denkbar ist auch eine bidirektionale Kommunikation. Hierzu wäre seitens der elektronisch gesteuerten Schutzeinrichtung 30 zusätzlich ein Sender vorzusehen, über den die Kommunikation zur Blendeinrichtung 1 bzw. deren Steuerungseinrichtung erfolgen kann.

Die Stromversorgung erfolgt über einen Energiespeicher 21, bei dem es sich bevorzugt um einen wiederaufladbaren Akkumulator handelt, der wie beschrieben über den Ladeanschluss 7 aufgeladen werden kann. Der Steuerungseinrichtung 16 ist der Schalter 6 zugeordnet, über den die Blendeinrichtung 1 aktiviert werden kann respektive der Zugangscode eingegeben werden kann, wenn es sich bei dem Schalter 6 um ein Tastenfeld oder Ähnliches handelt.

Des Weiteren ist wenigstens ein Abstandssensor 22 vorgesehen, wobei natürlich auch mehrere solcher Abstandssensoren in unterschiedlicher Position am Gehäuse 2 verbaut sein können. Über diese Abstandssensoren wird nach Verbringen der Blendeinrichtung 1 ein Abstand zu einem in der Nähe befindlichen Gegenstand erfasst. Insbesondere kann hierüber ein Annähern einer Person erfasst werden. Die Steuerungseinrichtung 16 empfängt das entsprechende gemessene Abstandssignal des Sensors 22. Ergibt sich, dass die Person in einem definierten Mindestabstand zur Blendeinrichtung 1 ist, kann die Steuerungseinrichtung 16 die Leuchtmittel 5 entsprechend ansteuern, um den Lichtpuls zu erzeugen. Denn dann ist sichergestellt, dass die zu irritierende Person in einer hinreichenden Mindestdistanz zur Blendeinrichtung 1 ist, und folglich die optimale Irritationswirkung erreichbar ist.

Schließlich ist eine Einrichtung 23 zur Selbstzerstörung vorgesehen, die beispielsweise über die externe übergeordnete Fernsteuerungseinrichtung per Funk angesteuert werden kann. Ist eine Blendeinrichtung 1 unwiederbringlich verloren, kann durch Ansteuerung der Selbstzerstörungseinrichtung 23, die beispielsweise einen kleinen Zünder umfasst, der dann detoniert, sichergestellt werden, dass die Blendeinrichtung 1 komplett inaktiv wird und nicht gegen einen selbst verwendet werden kann.

Die Kommunikation über die Kommunikationseinrichtung 18 erfolgt entweder optisch oder per Funk, in jedem Fall drahtlos, wozu der Empfänger 19 und der Sender 20 entsprechend ausgelegt sind respektive an entsprechender Position im Gehäuse 2 verbaut sind.

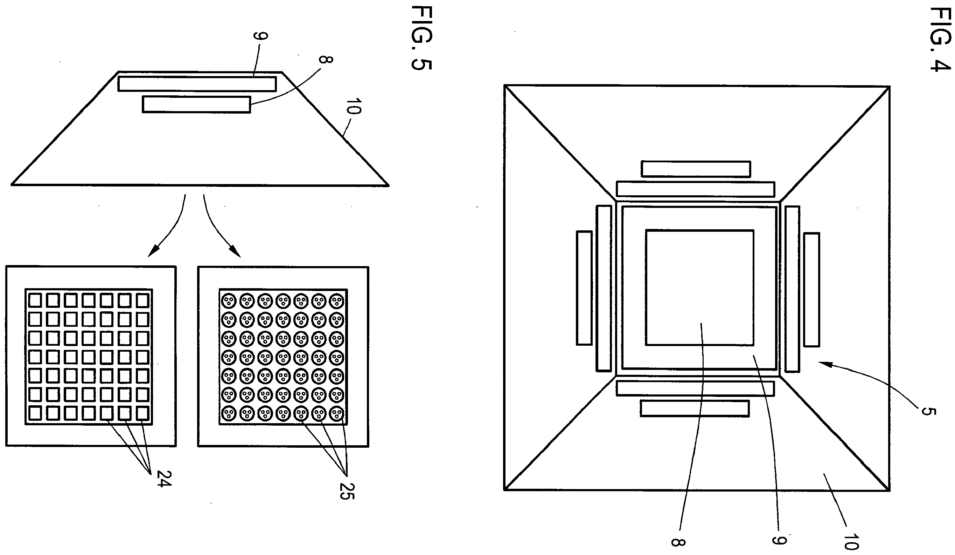

Fig. 4 zeigt eine Prinzipdarstellung des Leuchtmittels 5, hier in Form eines Laserdiode-Arrays 8, das samt dem Träger 9 in einem hier rechteckförmigen Reflektor 10 aufgenommen ist. Der Reflektor 10 weist, siehe Fig. 4, schräge Wandflächen auf. Er besteht aus Metall oder Kunststoff und weist an seinen Innenseiten entsprechende Spiegelflächen auf. Der Reflektorform folgend können wie beschrieben an den Gehäusehälften 3, 4 entsprechende Vertiefungen vorgesehen sein, in die die Reflektoren eingesetzt werden, um diese samt Leuchtmitteln 5 und den Linsen 12, worauf nachfolgend noch eingegangen wird, auf einfache Weise zu montieren.

Bei dem Laserdioden-Array 8 kann es sich, siehe Fig. 5, um ein Array bestehend aus farbiges, monochromes und kohärentes Licht emittierenden Laserdioden 24 handeln. Ersichtlich sind die Laserdioden 24 in einer möglichst dichten Packung im Array angeordnet.

Alternativ zur Verwendung von nur ein gemeinsames Farblicht emittierenden Laserdioden können auch RGB-Laserdioden-Cluster 25 das Array bilden. Jedes RGB-Laserdioden-Cluster 25 besteht aus drei Laserdioden, von denen eine erste rotes Licht, eine zweite grünes Licht und eine dritte blaues Licht emittiert. Es ist damit eine Farbmischung und damit die Erzeugung weißen Lichts möglich. Daneben können auch beliebige andere Lichtfarben gemischt werden.

Das Spektrum respektive der Spektralbereich des von dem Laserdioden-Array 8 emittierten Lichtes sollte zwischen 280 nm bis 1 mm liegen. Das heißt, dass der Spektralbereich zwischen UVB und fernem Infrarot liegt. In jedem Fall sollte der Spektralbereich des emittierten Lichts den Bereich des sichtbaren Lichtes erfassen, und sich bevorzugt in den Infrarotbereich erstrecken, da viele optische Geräte mit Infrarotsensoren arbeiten. Denkbar ist es auch, Laserdioden-Arrays nebeneinander anzuordnen, die unterschiedliche Spektralbereiche bedienen. Ein Beispiel hierfür zeigt Fig. 5. So kann ein erstes Laserdioden-Array 8 verbaut werden, das vorzugsweise monochromes Licht im sichtbaren Spektralbereich emittiert. Benachbart kann ein Laserdioden-Array 8 verbaut werden, das primär im Infrarotbereich emittiert. Auf diese Weise lässt sich ein extrem breitbandiger Spektralbereich abdecken.

Der Reflektor 10 ist, siehe Fig. 3, über die Linse 12 abgeschlossen. Diese ist im Reflektor beispielsweise verklebt. Die Linsenform entspricht der Reflektorform. Der Reflektor kann anstelle der in Fig. 4 gezeigten Rechteckform auch rund sein, entsprechend auch die Linse 12.

Über die Steuerungseinrichtung 16 respektive den Laserdioden-Treiber 15 sind sämtliche gehäuseseitig verbauten Leuchtmittel 5, also die Laserdioden-Arrays 8 separat ansteuerbar. Das oder die Leuchtmittel 5, die anzusteuern sind, richtet sich nach dem seitens der Steuerungseinrichtung 16 abzuarbeitenden Programm. Das heißt, dass letztlich beliebige Ansteuerabfolgen möglich sind, also eine synchrone Ansteuerung aller Laserdioden-Arrays 8, die Ansteuerung einzelner Laserdioden-Arrays 8, die gruppenweise Ansteuerung der Laserdioden-Arrays 8, die Intensitätsvariation, die Ansteuerung nur ausgewählter lokaler Gruppen in Abhängigkeit der Erfassung über den Abstandssensor 22 und ähnliches, wie natürlich auch entsprechende Wiederholungen ohne weiteres möglich sind, solange die Versorgung über den Energiespeicher 21 gewährleistet ist. Es können also unterschiedlichste Lichterzeugungsmuster emittiert werden.

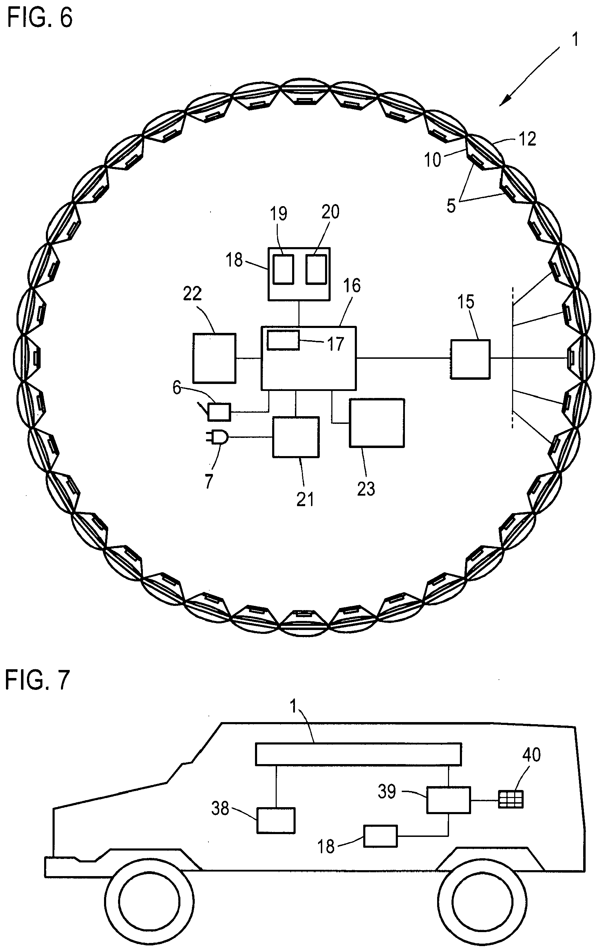

Fig. 6 zeigt eine weitere Ausgestaltung einer Blendeinrichtung 1, bei denen die Leuchtmittel 5 mit ihren Reflektoren 10 und Linsen 12 in Ringform angeordnet sind. Das heißt, dass das Gehäuse in diesem Fall ringförmig wäre respektive als dosenartige Scheibe ausgeführt wäre, anders als gemäß Fig. 2, in dem ein eckiges Gehäuse gezeigt ist. Eine gleiche Konfiguration wäre aber auch bei einer Kugelausführung des Gehäuses gegeben, auch dann wären die Leuchtmittel 5 in einer vergleichbaren Anordnung positioniert, jedoch über die gesamte Kugelfläche des Gehäuses verteilt. Wie bereits Fig. 5 nahelegt, ist eine Vielzahl an einzelnen Leuchtmitteln 5 nebst Reflektoren 10 und Linsen 12 am Gehäuse angeordnet, je nach Größe respektive Fläche des Gehäuses können 100 oder mehr solcher Leuchtmittel 5 verbaut sein, da insbesondere LED-Arrays bekanntlich sehr klein bauend sind, trotz der Möglichkeit, als Hochleistungs-LED Licht mit hoher Intensität emittieren zu können. Folglich ist eine extrem hohe Packungsdichte erreichbar, resultierend in der Möglichkeit, einen Lichtpuls mit extrem hoher Intensität respektive Helligkeit zu emittieren.

Eine solche ringförmige Blendeinrichtung 1 kann z. B. an einem UAV, z. B. einer Multicopter-Drohne angeordnet werden, über die die Blendeinrichtung 1 in das Zielgebiet geflogen wird. Sie kann entweder dort abgesetzt werden oder im Flug, also in definierter Höhe den oder die Lichtpulse emittieren. Über die integrierte Kommunikationseinrichtung 18 ist auch über größere Distanzen eine Synchronisation mit entsprechenden Schutzeinrichtungen 30 möglich.

Fig. 7 zeigt schließlich eine leistenförmige Blendeinrichtung 1, die an einem Kraftfahrzeug angeordnet ist. Die Blendeinrichtung 1 ist mit einer Batterie 38 des Kraftfahrzeugs verbunden und wird über diese betrieben. Ferner ist eine kraftfahrzeugseitige Steuerungseinrichtung 39 vorgesehen, über die der Betrieb der Blendeinrichtung 1 steuerbar ist. Ein Bediener kann z. B. über eine Eingabeeinrichtung 40 die entsprechende Programmierung der Blendeinrichtung 1 vornehmen. Über eine kraftfahrzeugseitige Kommunikationseinrichtung 18 kann die Synchronisation mit externen Schutzeinrichtungen 30, sofern eine solche notwendig ist, erfolgen.

### Bezugszeichenliste

- 1: Blendeinrichtung
- 2: Gehäuse
- 3: Halbschale
- 4: Halbschale
- 5: Leuchtmittel
- 6: Schalter
- 7: Ladeanschluss
- 8: LED-Array
- 9: Träger
- 10: Reflektor
- 11: Element
- 12: Linse
- 13: Versorgungsleitung
- 14: Steckverbinder
- 15: LED-Treiber
- 16: Steuerungseinrichtung
- 17: Timer
- 18: Kommunikationseinrichtung
- 19: Empfänger
- 20: Sender
- 21: Energiespeicher
- 22: Abstandssensor
- 23: Einrichtung
- 24: LED
- 25: RGB-LED-Cluster
- 30: Schutzeinrichtung
- 31: Schutzbrille
- 32: Shutterbrille
- 33: Filter
- 34: Gerätschaft
- 35: Schutzeinrichtung
- 36: Steuerelektronik
- 37: Empfänger
- 38: Batterie
- 39: Steuerungseinrichtung
- 40: Eingabeeinrichtung

## Patentansprüche

1. Lichtoptische Irritationseinrichtung, umfassend eine Blendeinrichtung (1) mit einem oder mehreren Leuchtmitteln (5) und eine Steuerungseinrichtung (16), wobei das oder die Leuchtmittel (5) zur Emission eines oder mehrerer Lichtpulse mit einer bekannten Frequenz oder einem bekannten Frequenzband ansteuerbar ist oder sind,
sowie wenigstens eine Schutzeinrichtung (30, 31, 32, 33, 35), die von einer durch den Lichtpuls irritierbaren Person tragbar oder an einer durch den Lichtpuls irritierbaren Gerätschaft (34) anbringbar oder integriert ist, und die eine Filtereinrichtung (33), die auf die Frequenz oder das Frequenzband des Lichtpulses und/oder die Gabefrequenz der Lichtpulse abgestimmt ist, umfasst.

2. Irritationseinrichtung nach Anspruch 1,
**dadurch gekennzeichnet,**
**dass** die Frequenz oder das Frequenzband des Lichtpulses und damit auch die Filterfrequenz oder das Filterfrequenzband der Filtereinrichtung (33) im Bereich von 280 nm bis 1 mm liegt.

3. Irritationseinrichtung nach Anspruch 1 oder 2,
**dadurch gekennzeichnet,**
**dass** Leuchtmittel (5) auf Halbleiterbasis vorgesehen sind.

4. Irritationseinrichtung nach Anspruch 3,
**dadurch gekennzeichnet,**
**dass** die Leuchtmittel (5) LEDs oder Laserdioden oder Laser sind.

5. Irritationseinrichtung nach Anspruch 4,
**dadurch gekennzeichnet,**
**dass** die Leuchtmittel (5) jeweils drei LEDs oder Laserdioden umfassende RGB-Cluster sind.

6. Irritationseinrichtung nach einem der vorangehenden Ansprüche,
**dadurch gekennzeichnet,**
**dass** mehrere Lichtpulse hintereinander emittierbar sind, wobei die Pulsgabefrequenz und/oder die Pulsdauer einstellbar ist.

7. Irritationseinrichtung nach einem der vorangehenden Ansprüche,
**dadurch gekennzeichnet,**
**dass** bei Verwendung mehrerer Leuchtmittel (5) die einzelnen Leuchtmittel (5) Licht mit zueinander unterschiedlichen Frequenzen oder Frequenzbändern emittieren.

8. Irritationseinrichtung nach einem der vorangehenden Ansprüche,
**dadurch gekennzeichnet,**
**dass** die Frequenz oder das Frequenzband des Lichtpulses fest vorgegeben oder einstellbar ist.

9. Irritationseinrichtung nach einem der vorangehenden Ansprüche,
**dadurch gekennzeichnet,**
**dass** die Filterfrequenz oder das Filterfrequenzband fest vorgegeben oder einstellbar ist.

10. Irritationseinrichtung nach einem der vorangehenden Ansprüche,
**dadurch gekennzeichnet,**
**dass** die Schutzeinrichtung (30, 31, 32, 33, 35) eine elektronisch steuerbare Filtereinrichtung (33) aufweist.

11. Irritationseinrichtung nach Anspruch 8 oder 9 und Anspruch 10,
**dadurch gekennzeichnet,**
**dass** die steuerbare Filtereinrichtung (33) und die Steuerungseinrichtung (16) der Blendeinrichtung (1) miteinander synchronisiert sind.

12. Irritationseinrichtung nach Anspruch 11,
**dadurch gekennzeichnet,**
**dass** die Schutzeinrichtung (30, 31, 32, 33, 35) und die Blendeinrichtung (1) zur Synchronisation drahtlos miteinander kommunizieren.

13. Irritationseinrichtung nach einem der vorangehenden Ansprüche,
**dadurch gekennzeichnet,**
**dass** die Blendeinrichtung (1) eine Blendgranate ist oder eine lineare oder bogenförmige oder ringförmige Geometrie umfassend mehrere Leuchtmittel (5) aufweist.

14. Irritationseinrichtung nach einem der vorangehenden Ansprüche,
**dadurch gekennzeichnet,**
**dass** die Leuchtmittel (5) in Form separater Leuchtmittel-Arrays (8) vorgesehen sind, wobei an oder in einem Gehäuse (2) mehrere Arrays verteilt angeordnet sind.

15. Irritationseinrichtung nach einem der vorangehenden Ansprüche,
**dadurch gekennzeichnet,**
**dass** den Leuchtmitteln (5), insbesondere den Arrays (8), optische Elemente (10, 11, 12) zur Beeinflussung des emittierten Lichts zugeordnet sind, insbesondere Linsen, Prismen oder feste oder bewegliche Spiegel vorgesehen sind.

16. Irritationseinrichtung nach einem der vorangehenden Ansprüche,
**dadurch gekennzeichnet,**
**dass** im Gehäuse (2) eine Kommunikationseinrichtung (18) umfassend zumindest einen mit der Steuerungseinrichtung (16) kommunizierenden Empfänger (19) zum Empfangen von von einem externen Sender (20) gegebenen Steuersignalen oder Programmierinformationen vorgesehen ist.

17. Irritationseinrichtung nach Anspruch 16,
**dadurch gekennzeichnet,**
**dass** die Kommunikationseinrichtung (18) ferner einen mit der Steuerungseinrichtung (16) kommunizierenden Sender (20) zum Aussenden von Signalen umfasst.

18. Irritationseinrichtung nach einem der vorangehenden Ansprüche,
**dadurch gekennzeichnet,**
**dass** im Gehäuse (2) ein Energiespeicher (21), insbesondere ein wiederaufladbarer Energiespeicher vorgesehen ist, wobei ein wiederaufladbarer Energiespeicher dem Gehäuse (2) entnehmbar ist, oder wozu am Gehäuse (2) ein Ladeanschluss (7) vorgesehen ist.

19. Blendgranate nach einem der vorangehenden Ansprüche,
**dadurch gekennzeichnet,**
**dass** ein den Betrieb freischaltender, bedienerseitig zu betätigender Schalter (6) vorgesehen ist.

20. Irritationseinrichtung nach einem der vorangehenden Ansprüche,
**dadurch gekennzeichnet,**
**dass** das Gehäuse (2) der Blendeinrichtung (1) aus zwei miteinander verbindbaren, einen Hohlraum umschließenden kugel- oder napfförmigen Halbschalen (3, 4) besteht, an denen die Leuchtmittel (5), insbesondere die Arrays (8) angeordnet sind, wobei die schalenseitigen Leuchtmittel (5), insbesondere die Arrays (8) über jeweils eine gemeinsame Steckerverbindung (14) mit der Steuerungseinrichtung (16) oder einem Treiber (15) verbindbar sind.
